# EUROPÄISCHE PATENTSCHRIFT

(11) **EP 1 589 019 B1**
(45) Veröffentlichungstag und Bekanntmachung des Hinweises auf die Patenterteilung: **13.08.2008**
(21) Anmeldenummer: 04009280.1
(22) Anmeldetag: 20.04.2004
(51) Int. Cl.: C07D 495/04, C07D 333/20, C07C 211/08, C07C 209/68

(54) **Stereoselektives Verfahren zur Herstellung von Clopidogrel**
Stereoselective process for the preparation of Clopidogrel
Procédé de préparation stéréosélective de Clopidogrel

(43) Veröffentlichungstag der Anmeldung: 26.10.2005
(73) Patentinhaber: Ratiopharm GmbH, 89079 Ulm (DE)
(72) Erfinder: Stohandl, Jiri, 236 (CZ); Frantisek, Jaroslav, 618 00 Brno (CZ); Ness, Winfried, 89077 Ulm (CZ)
(74) Vertreter: Best, Michael

(56) Entgegenhaltungen:
- WO-A-99/18110
- WO-A-03/035652
- M.M-L. CHAM AND J.B. ROBINSON: "Stereoisomeric Lactoyl-beta-methylcholine Iodides. Interaction with Cholinesterase and Acetylcholinesterase" J. MED. CHEM., Bd. 17, Nr. 10, 1974, Seiten 1057-60, XP002297328

## Beschreibung

(R)-1-(Dimethylamino)-2-propanol ((R)-Dimepranol) ist eine bevorzugte Ausgangsverbindung in dem in der EP-A 1589019 beschriebenen Verfahren zur Herstellung von Clopidogrel. M.M.-L. CHAM und J.B. ROBINSON offenbaren in J. MED. CHEM., Bd. 17, Nr. 10, 1974, Seiten 1057-60 ein Verfahren zur Herstellung von (R)-Dimepranol, bei dem der racemische Aminoalkohol (±)-1-Dimethylaminopropan-2-ol unter Verwendung von D-(+)-Weinsäure durch Umsetzung und nachfolgende Rekristallisation dargestellt werden kann.

Erfindungsgemäß wurde gefunden, dass (R)-Dimepranol auch auf einfache Art und Weise durch optische Auflösung des entsprechenden racemischen Aminoalkohols mit Di-O-Benzoyl-L-(-)-weinsäure erhalten werden kann. Dies erhöht die Wirtschaftlichkeit des Verfahrens zur Herstellung von Clopidogrel.

Der optisch aktive Aminoalkohol (R)-Dimepranol ist zwar kommerziell erhältlich, erfindungsgemäß wurde jedoch ein Verfahren gefunden, mit dem der optisch aktive Aminoalkohol leicht und kostengünstig aus dem racemischen Aminoalkohol hergestellt werden kann. Hierzu werden 0,5 Äquivalente Dibenzoyl-L-weinsäure mit einem Äquivalent des racemischen Alkohols in einem geeigneten Lösemittel, wie einem C₁-C₄-Alkanol, insbesondere Ethanol, gelöst. Hierbei bildet sich das entsprechende Dibenzoyl-L-weinsäurederivat des R(-)Dimepranols. Die Lösung wird dann mit einer achiralen Mineralsäure, z.B. Salzsäure, angesäuert. Hierdurch bildet sich das Salz (z.B. das Hydrochloridsalz) mit dem entgegengesetzten Enantiomer des Dimepranols, dem S(+)Dimepranol, das dadurch in Lösung gehalten wird. Gegebenenfalls nach Animpfen fällt das Dibenzoyl-L-weinsäurederivat des R(-)Dimepranols als kristallines Produkt aus, während das Salz des S(+)Dimeparanols in Lösung bleibt. Aus dem ausgefallenen Salz kann die freie (R)-Dimepranolbase durch Umsetzung mit einer geeigneten Base, wie Natrium- oder Kaliumhydroxid, in einem geeigneten Lösemittel, wie einem Alkohol, insbesondere in Ethanol, auf an sich bekannte Art und Weise erhalten werden. In einer alternativen Ausführungsform kann z.B. auch das Dibenzoyl-L-weinsäurederivat des R-Dimepranols durch Behandlung mit trockenem Chlorwasserstoff in das R-Dimepranol-Hydrochlorid überführt werden, das als solches oder nach Behandlung mit einer geeigneten Base in der basischen Form weiterverwendet werden kann.

Durch das erfindungsgemäße Verfahren wird die Menge an benötigter Dibenzoyl-L-weinsäure reduziert, was das Verfahren sehr wirtschaftlich macht. Ferner hat das erhaltene Produkte eine hohe optische Reinheit, und mehrfache Rekristallisationen sind in der Regel nicht erforderlich.

Das folgende Beispiel erläutert die Erfindung.

### Beispiel 1

### (R)-1-(Dimethylamino)-2-propanol ((R)-Dimepranol)

Eine Lösung aus 190 g Dibenzoyl-L-weinsäure in 1200 ml Ethanol wurde mit 103 g 1-(Dimethylamino)-2-propanol gemischt. Die entstandene Lösung wurde mit 36 ml 36%-iger Salzsäure angesäuert und angeimpft. Nachdem es über Nacht stehengelassen wurde, wurde das kristalline Produkt abfiltriert, mit kaltem Ethanol und Diethylether gewaschen und getrocknet. Rohes (R)-1-(Dimethylamino)-2-propanoldibenzoyl-L-tartrat wurde aus 2700 ml heißem Ethanol umkristallisiert, und die Ausbeute betrug 187 g reines diastereomeres Salz. Das Salz wurde in 1000 ml kalter 20%-iger Natriumhydroxidlösung gelöst und in Dichlormethan extrahiert. Der Extrakt wurde getrocknet, filtriert, abgedampft, und das Restöl wurde durch Destillation bei Atmosphärendruck gereinigt. Das Produkt destillierte bei 122 - 124°C ab, und die Ausbeute betrug 46 g (R)-Dimepranol (45% bezogen auf das Ausgangsracemat), [α_{D}²⁰] -27°.

## Patentansprüche

1. Verfahren zur Herstellung der Verbindung 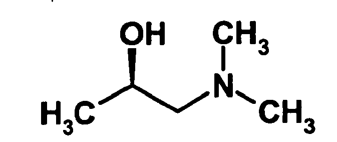 bei dem eine Gemisch der Verbindungen 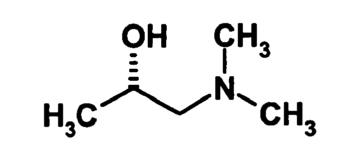 und 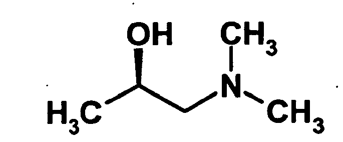 mit L-(-)-Di-O-benzoyl-L-(-)-Weinsäure (L-(-)-DBTA) umgesetzt und das L-(-)-DBTA-Salz der Verbindung 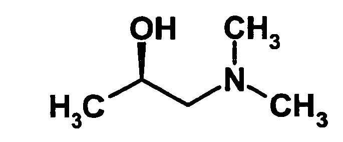 abgetrennt wird.

## Claims

1. Process for the preparation of the compound 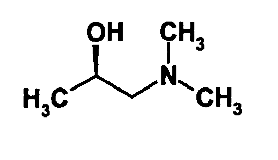 wherein a mixture of the compounds 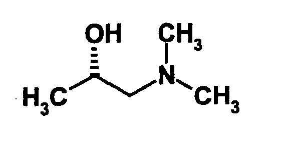 and 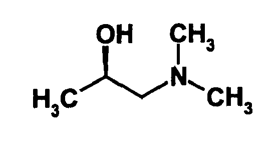 is treated with L-(-)-di-O-benzoyl-L-(-)-tartaric acid (L-(-)-DBTA) and the L-(-)-DBTA salt of the compound 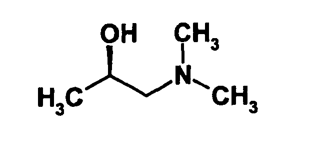 is separated.

## Revendications

1. Procédé de fabrication du composé suivant : 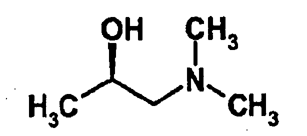 dans lequel on fait réagir un mélange des composés : 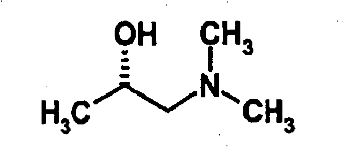 et 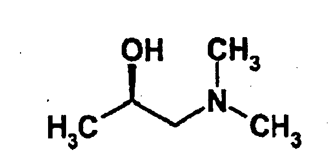 avec l'acide L-(-)-di-O-benzoyl-L-(-)-tartrique (L-(-)-DBTA) et on sépare le sel de L-(-)-DBTA du composé :
